# EUROPEAN PATENT SPECIFICATION

(11) **EP 1 925 328 B1**
(45) Date of publication and mention of the grant of the patent: **22.07.2015**
(21) Application number: 08002511.7
(22) Date of filing: 07.06.2006
(51) Int. Cl.: A61M 1/36, B04B 5/04, B04B 9/14, B04B 13/00

(54) **Set of bags for the separation of a composite liquid in a centrifuge**
Beutelset zur Auftrennung einer Flüssigkeit aus mehreren Komponenten in einer Zentrifuge
Ensemble poche permettant de séparer un liquide composite utilisant une centrifugeuse

(30) Priority: 22.06.2005 US 693320 P
(43) Date of publication of application: 28.05.2008
(62) Divisional of application: 06076188.9
(73) Proprietor: Terumo BCT, Inc., Lakewood, CO 80215 (US)
(72) Inventor: Hudock, Darryl, Highlands Ranch Colorado 80215 (US); Joseph, Daniel A., Golden Colorado 80403 (US); Dolecek, Victor D., Englewood Colorado 80111 (US); Hlavinka, Dennis J., Arvada Colorado 80007 (US)
(74) Representative: Roberts, Mark Peter

(56) References cited:
- EP-A- 0 536 594
- EP-A- 0 578 086
- WO-A-00/54823
- US-A1- 2003 191 005
- US-B1- 6 652 475

## Description

The present invention relates to a set of bags for use with an apparatus for separating at least two discrete volumes of composite liquid into at least two components.

The apparatus and a method are particularly appropriate for the separation of biological fluids comprising an aqueous component and one or more cellular components. For example, potential uses of the invention indude: extracting a plasma component and a cellular component (including platelets, white blood cells, and red blood cells) from a volume of whole blood, the cellular component being subsequently filtered so as to remove platelets and white blood cells from the red blood cells; extracting a plasma component, in which a substantial amount of platelets is suspended, and a red blood cell component from a volume of whole blood, the white blood cells being subsequently removed by filtration from the platelet component and the red blood cell component; extracting a plasma component, a platelet component, and a red blood cell component from a volume of whole blood, the white blood cells being subsequently removed by filtration from the platelet component and the red blood cell component

An apparatus for processing blood components is known from document WO 03/089027. This apparatus comprises a centrifuge adapted to cooperate with an annular separation bag connected to at least one product bag, e.g. a platelet component bag. The centrifuge includes:
- a rotor having a turntable for supporting the separation bag, and a central compartment for containing the product bag connected to the separation bag; and
- a squeezing system for squeezing the separation bag and causing the transfer of a separated component (e.g. platelets suspended in plasma) from the separation bag into the product bag.

With this apparatus, a single discrete volume of blood is processed at once.

It is desirable to design a separation apparatus that can process at once at least two discrete volumes of a composite liquid, in particular discrete volumes that may be not the same, and with the proportions of the various components of the composite liquid that may vary from one discrete volume to another one.

US 6,652,475 discloses an arrangement where two satellite bags connect to a separation bag at the same location, with the two connection points being equidistant from the rotation axis.

The invention is defined in claim 1. Other preferable features are as follows:
- The bag set further comprises a third tube having a first end connected to the tip of the collection and separation bag and a second end connected to the inlet channel.

The third tube comprises a detection section for engaging a cell detector of the centrifuge.
- The bag set further comprises a breakable stopper connected to the third tube.
- The bag set is for the separation of whole blood into a plasma component, a platelet component and a red blood cell component, and the first satellite bag is for collecting the plasma component and the second satellite bag is for collecting the platelet component.
- The bag set further comprises:
- a third satellite bag for collecting red blood cells;
- a third tube having:
   - a first section having a first end connected to the top of the collection and separation bag; and
   - a second section having a second end connected to the third satellite bag; and
- a leuko-reduction filter having an inlet connected to a second end of the first section of the third tube and an outlet connected to a first end of the second section of the third tube.
- The bag set further comprises a needle connected to a second end of the collection tube.

Other features and advantages of the invention will appear from the following description and accompanying drawings, which are to be considered exemplary only.

In the accompanying drawings:
Figure 1 is a schematic view of a first set of bags designed for cooperating with a separation apparatus;
Figure 2 is a schematic view of a second set of bags designed for cooperating with a separation apparatus;
Figures 3a, 3b are schematic views of two variants of a detail of the set of bags of figure 2;
Figure 4 is a schematic view, partly in cross-section along a diametral plane, of a separation apparatus;
Figure 5 is a top view of the rotor of the separation apparatus of figure 4;
Figure 6 is a perspective view of a first embodiment of a passive balancing unit for a separation apparatus;
Figure 7 is a perspective view of a passive balancing unit for a separation apparatus;
Figure 8 is schematic view, in cross-section along a radial plane, of a separation cell of the separation apparatus of figures 4 and 5;
Figure 9 is schematic view, in cross-section along a radial plane, of a separation cell adjacent to a storage container;
Figure 10 is a perspective view of a rotor of a separation apparatus;
Figure 11 is a cross-section view of the rotor of figure 10, along a diametral plane;
Figure 12 is a top view of the rotor of figure 10;
Figure 13 is a schematic view, in cross-section along a diametral plane, of a separation apparatus;
Figure 14 is schematic view, in cross-section along a radial plane, of a separation cell of the separation apparatus of figure 13;
Figure 15 is a perspective view of the flexible diaphragm of the separation cell of figure 14;
Figures 16 to 18 are schematic views, in cross-section along a radial plane, of the separation cell figure 14 containing a separation bag at different stages of a separation process; and
Figure 19 is a schematic view, in cross-section along a diametral plane, of a separation apparatus.

For the sake of clarity, the invention will be described with respect to a specific use, namely the separation of whole blood into at least two components, in particular into a plasma component and a red blood cell component, or into a plasma component, a platelet component and a red blood cell component. The discrete volume mentioned hereunder will typically be the volume of a blood donation. The volume of a blood donation may vary from one donor to another one (450ml plus or minus 10%). It is also recalled that the proportion of the components of blood usually varies from one donor to another one, in particular the hematocrit, which is the ratio of the volume of the red blood cells to the volume of the sample of whole blood considered. In other words the density of blood may slightly vary for one donor to another one. It should be understood however that this specific use is exemplary only.

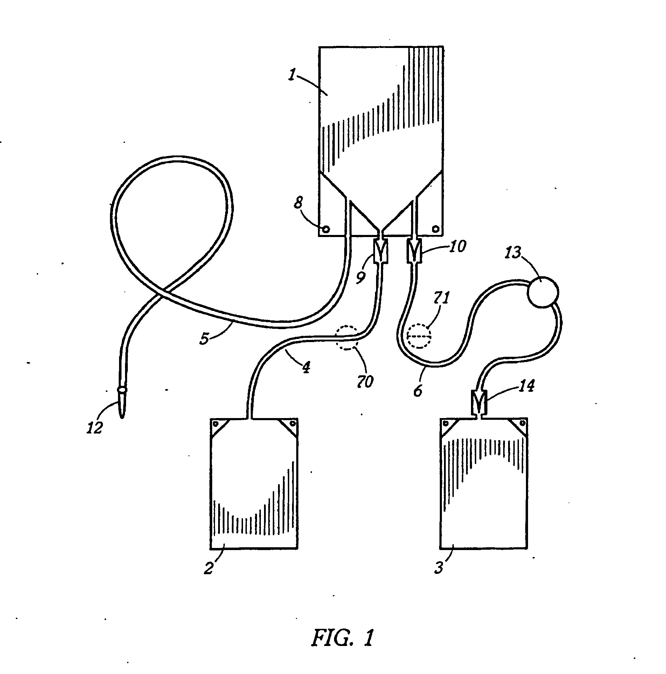

Figure 1 shows an example of a set of bags adapted to the separation of a composite liquid (e.g. whole blood) into a first component (e.g. a plasma component containing or not a substantial amount of suspended platelets) and a second component (e.g. a blood cell component). This bag set comprises a flexible separation bag 1 and two flexible satellite bags 2, 3 connected thereto.

When the composite liquid is whole blood, the separation bag 1 has two purposes, and is successively used as a collection bag and as a separation bag. It is intended for initially receiving a discrete volume of whole blood from a donor (usually about 450 ml) and to be used later as a separation chamber in a separation apparatus. The separation bag 1 is flat and generally rectangular. It is made of two rectangular sheets of plastic material that are welded together so as to define therebetween an interior space having a main rectangular portion connected to a triangular top downstream portion. A first tube 4 is connected to the tip of the triangular portion, and a second and a third tubes 5, 6 are connected to either lateral edges of the triangular portion, respectively. The proximal ends of the three tubes 4, 5, 6 are embedded between the two sheets of plastic material so as to be parallel. The separation bag 1 further comprises a hole 8 in each of its corners that are adjacent to the three tubes 4, 5, 6. The holes 8 are used to secure the separation bag to a separation cell, as will be described later.

The separation bag initially contains a volume of anti-coagulant solution (typically about 63 ml of a solution of citrate phosphate dextrose for a blood donation of about 450 ml), and the first and third tubes 4, 6 are fitted at their proximal end with a breakable stopper 9, 10 respectively, blocking a liquid flow therethrough.

The second tube 5 is a collection tube having a needle 12 connected to its distal end. At the beginning of a blood donation, the needle 12 is inserted in the vein of a donor and blood flows into the collection (separation) bag 1. After a desired volume of blood has been collected in the collection (separation) bag 1, the collection tube 5 is sealed and cut.

The first satellite bag 2 is intended for receiving a plasma component. It is flat and substantially rectangular. It is connected to the distal end of the first tube 4.

The second satellite bag 3 is intended for receiving a red blood cell component. It is flat and substantially rectangular. It is connected to the distal end of the third tube 6. The third tube 6 comprises two segments respectively connected to the inlet and the outlet of a leuko-reduction filter 13. The second satellite bag 3 contains a volume of storage solution for red blood cells, and the third tube 6 is fitted at its distal end with a breakable stopper 14 blocking a liquid flow therethrough.

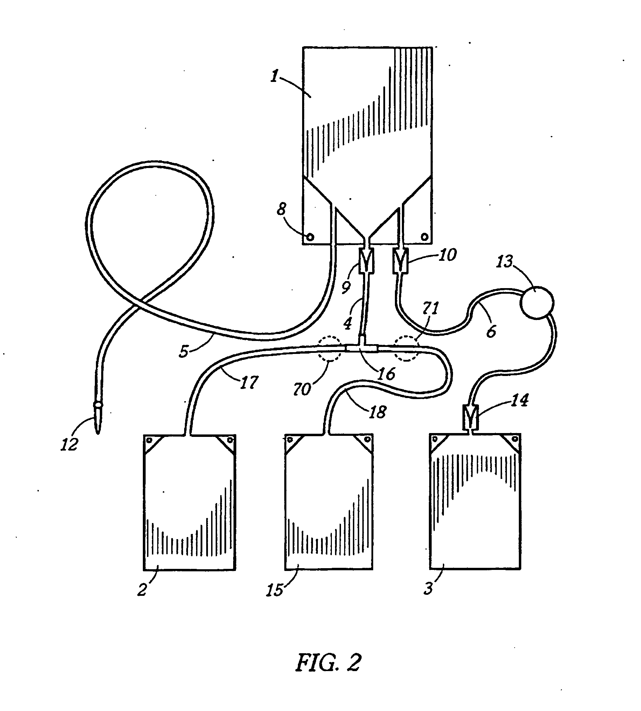

Figure 2 shows an example of a set of bags adapted to the separation of a composite liquid (e.g. whole blood) into a first component (e.g. a plasma component), an intermediate component (e.g. a platelet component), and a second component (e.g. a red blood cell component). This bag set comprises a flexible separation bag 1 and three flexible satellite bags 2, 3, 15 connected thereto.

This second set of bags differs from the set of bags of figure 1 in that it comprises a third satellite bag 15, which is intended to receive a platelet component, and a T-shaped three-way connector 16 having its leg connected by the first tube 4 to the separation bag 1, a first arm connected by a fourth tube 17 to the first satellite bag 2 (plasma component bag), and a second arm connected by a fifth tube 18 to the third satellite bag 15 (platelet component bag). Like the first and second satellite bags 2, 3, the third satellite bag 15 is flat and substantially rectangular.

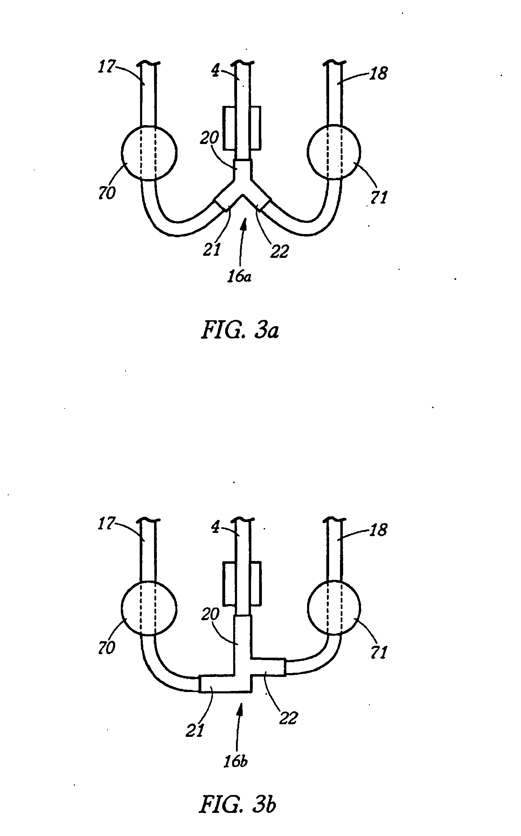

Figures 3a, 3b show two variants of the T-shaped three-way connector 16 of the bag set of figure 2.

The three-way connector 16a shown in figure 3a has the shape of a regular three-point star having a first outlet channel 21 and a second outlet channel 22 that are connected to an inlet channel 20 at an angle of about 120 degrees.

The three-way connector 16b shown in figure 3b, defines a first outlet channel 21 and a second outlet channel 22 that are perpendicularly connected to an inlet channel 20 and are offset along the inlet channel 20 so that the first outlet channel 21 is further than the second outlet channel 22 from the end of the inlet channel 20 that is connected to the first tube 4.

The three-way connectors 16, 16a, 16b are arranged such that when the separation bag of figure 2 (or any of its variants represented in figure 3a, 3b) is mounted in a separation apparatus (to be described in detail below), in which a separation cell for a separation bag 1, a storage container for the satellite bags 2, 3, 15, and a first and second pinch valve members for allowing or stopping a flow of liquid in the fourth and fifth tubes 17, 18 are arranged in this order along a radial direction from a rotation axis of the separation apparatus, with the pinch valve members being the closest to the rotation axis. In this particular configuration, when the fourth and fifth tubes 17, 18 are engaged in the first and second pinch valve members as shown in figures 2, 3a, 3b, then the three-way connector 16, 16b, or a bend in the fourth and fifth tubes 17, 18 in the case of the connector of figure 3a, are the closest portion(s) of the whole bag set to the rotation axis. The results of this disposition are that, when the separation apparatus rotates, any air in the bag set will gather in the connector in an area that is the closest to the rotation axis (junction point of the three channels 20, 21, 22 in the connectors shown in figures 2, 3b) or in the bends in the fourth and fifth tube 17, 18 between the connector and the pinch valve members 17, 18 when the connector used is the connector of figure 3a. This air buffer between the separation bag and the satellite bag will prevent any undesirable siphoning of contents of a satellite bag into the separation bag under centrifugation forces.

The three-way connector 16b presents a particular interest when the bag set of figure 2 is used to separate a plasma component and a platelet component. When the plasma component has been transferred into the first satellite bag 2 and the platelet component has been transferred into the third satellite bag 15, the connector 16b shown in figure 3b allow for flushing the second channel 22, which may contain remaining platelets, with a small volume of plasma trapped in the fourth tube 17 between the connector 16b and the first pinch valve member.

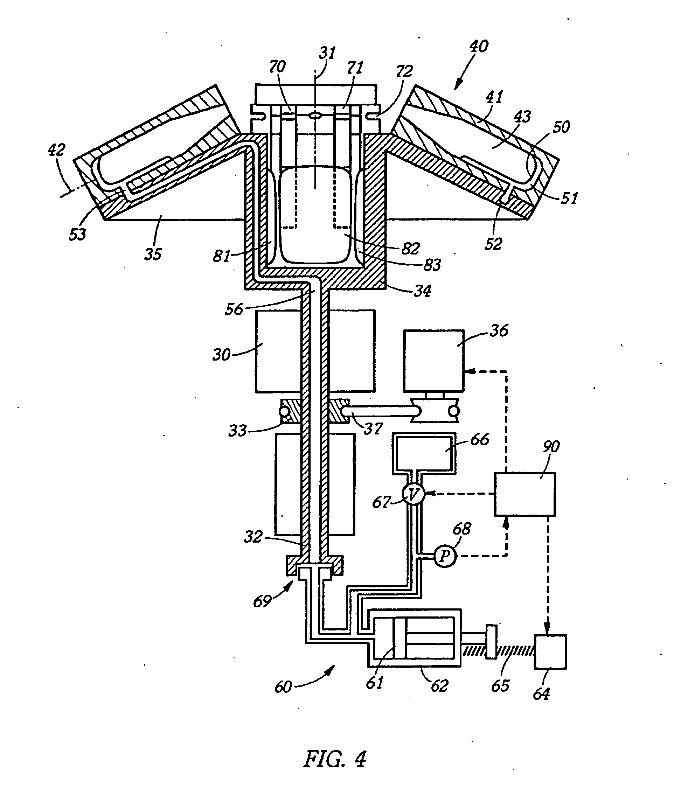

Figures 4, 5, 6, 8 show a first example an apparatus for simultaneously separating by centrifugation four discrete volumes of a composite liquid. The apparatus comprises:
- a centrifuge adapted to receive four of either set of bags shown in figures 1 and 2, with the four discrete volumes of a composite liquid contained in the four separation bags;
- a component transferring means for transferring at least one separated component from each separation bag into a satellite bag connected thereto;
- a first balancing means for initially balancing the rotor when the weights of the four separation bags are different; and
- a second balancing means for balancing the rotor when the weights of the separated components transferred into the satellite bags cause an unbalance of the rotor.

The centrifuge comprises a rotor that is supported by a bearing assembly 30 allowing the rotor to rotate around a rotation axis 31. The rotor comprises:
- a cylindrical rotor shaft 32 to which a pulley 33 is connected;
- a storage means comprising a central cylindrical container 34 for containing satellite bags, which is connected to the rotor shaft 32 at the upper end thereof so that the longitudinal axis of the rotor shaft 32 and the longitudinal axis of the container 34 coincide with the rotation axis 31, and
- a frusto-conical turntable 35 connected to the upper part of the central container 34 so that its central axis coincides with the rotation axis 31. The frusto-conical turntable 35 flares underneath the opening of the container 34. Four identical separation cells 40 are mounted on the turntable 35 so as to form a symmetrical arrangement with respect to the rotation axis 31.

The centrifuge further comprises a motor 36 coupled to the rotor by a belt 37 engaged in a groove of the pulley 33 so as to rotate the rotor about the rotation axis 31.

Each separation cell 40 comprises a container 41 having the general shape of a rectangular parallelepiped. The separation cells 40 are mounted on the turntable 35 so that their respective median longitudinal axes 42 intersect the rotation axis 31, so that they are located substantially at the same distance from the rotation axis 31, and so that the angles between their median longitudinal axes 42 are substantially the same (i.e. 90 degrees). The exact position of the separation cells 40 on the turntable 35 is adjusted so that the weight on the turntable is equally distributed when the separation cells 40 are empty, i.e. so that the rotor is balanced. It results from the arrangement of the separating cells 40 on the turntable 35 that the separating cells 40 are inclined with respect to the rotation axis 31 of an acute angle equal to the angle of the frustum of a cone that geometrically defines the turntable 35.

Each container 41 comprises a cavity 43 that is so shaped and dimensioned as to loosely accommodate a separation bag 1 full of liquid, of the type shown in figures 1 and 2. The cavity 43 (which will be referred to later also as the "separation compartment") is defined by a bottom wall, that is the farthest to the rotation axis 31, a lower wall that is the closest to the turntable 35, an upper wall opposite to the lower wall, and two lateral walls. The cavity 43 comprises a main part, extending from the bottom wall, which has substantially the shape of a rectangular parallelepiped with rounded angles, and an upper part, which has substantially the shape of a prism having convergent triangular bases. In other words, the upper part of the cavity 43 is defined by two couples of opposite walls converging towards the central median axis 42 of the cavity 43. One interest of this design is to cause a radial dilatation of the thin layer of a minor component of a composite fluid (e.g. the platelets in whole blood) after separation by centrifugation, and makes it more easily detectable in the upper part of a separation bag. The two couples of opposite walls of the upper part of the separation cell 40 converge towards three cylindrical parallel channels 44, 45, 46, opening at the top of the container 41, and in which, when a separation bag 1 is set in the container 41, the three tubes 4, 5, 6 extend.

The container 41 also comprises a hinged lateral lid 47, which is comprised of an upper portion of the external wall of the container 41, i.e. the wall that is opposite to the turntable 35. The lid 47 is so dimensioned as to allow, when open, an easy loading of a separation bag 1 full of liquid into the separation cell 40. The container 41 comprises a fast locking means (not shown) by which the lid 47 can be locked to the remaining part of the container 41.

The container 41 also comprises a securing means for securing a separation bag 1 within the separation cell 40. The bag securing means comprises two pins 48 protruding on the internal surface of the lid 47, close to the top of separation cell 40, and two corresponding recesses 49 in the upper part of the container 41. The two pins 48 are so spaced apart and dimensioned as to fit into the two holes 8 in the upper corner of a separation bag 1.

The separation apparatus further comprises a component transferring means for transferring at least one separated component from each separation bag into a satellite bag connected thereto. The component transferring means comprises a squeezing system for squeezing the separation bags 1 within the separation compartments 43 and causing the transfer of separated components into satellite bags 2, 3, 15.

The squeezing system comprises a flexible diaphragm 50 that is secured to each container 41 so as to define an expandable chamber 51 in the cavity thereof. More specifically, the diaphragm 50 is dimensioned so as to line the bottom wall of the cavity 43 and a large portion of the lower wall of the cavity 43, which is the closest to the turntable 35.

The squeezing system further comprises a peripheral circular manifold 52 that forms a ring within the turntable 35 extending close to the periphery of the turntable 35. Each expansion chamber 51 is connected to the manifold 52 by a supply channel 53 that extends through the wall of the respective container 41, close to the bottom thereof.

The squeezing system further comprises a hydraulic pumping station 60 for pumping a hydraulic liquid in and out the expandable chambers 51 within the separation cells 40. The hydraulic liquid is selected so as to have a density slightly higher than the density of the more dense of the components in the composite liquid to be separated (e.g. the red blood cells, when the composite liquid is blood). As a result, during centrifugation, the hydraulic liquid within the expandable chambers 51, whatever the volume thereof, will generally remain in the most external part of the separation cells 40. The pumping station 60 is connected to the expandable chambers 51, through a rotary seal 69, by a duct 56 that extends through the rotor shaft 32, the bottom and lateral wall of the central container 34, and, from the rim of the central container 34, radially through the turntable 35 where it connects to the manifold 52.

The pumping station 60 comprises a piston pump having a piston 61 movable in a hydraulic cylinder 62 fluidly connected via a rotary fluid coupling 63 to the rotor duct 54. The piston 61 is actuated by a stepper motor 64 that moves a lead screw 65 linked to the piston rod. The hydraulic cylinder 62 is also connected to a hydraulic liquid reservoir 66 having an access controlled by a valve 67 for selectively allowing the introduction or the withdrawal of hydraulic liquid into and from a hydraulic circuit including the hydraulic cylinder 62, the rotor duct 56 and the expandable hydraulic chambers 51. A pressure gauge 68 is connected to the hydraulic circuit for measuring the hydraulic pressure therein.

The separation apparatus further comprises four pairs of a first and second pinch valve members 70, 71 that are mounted on the rotor around the opening of the central container 34. Each pair of pinch valve members 70, 71 faces one separation cell 40, with which it is associated. The pinch valve members 70, 71 are designed for selectively blocking or allowing a flow of liquid through a flexible plastic tube, and selectively sealing and cutting a plastic tube. Each pinch valve member 70, 71 comprises an elongated cylindrical body and a head having a groove 72 that is defined by a stationary upper jaw and a lower jaw movable between an open and a closed position. The groove 72 is so dimensioned that one of the tubes 4, 17, 18 of the bag sets shown in figures 1 and 2 can be snuggly engaged therein when the lower jaw is in the open position. The elongated body contains a mechanism for moving the lower jaw and it is connected to a radio frequency generator that supplies the energy necessary for sealing and cutting a plastic tube. The pinch valve members 70, 71 are mounted inside the central container 34, adjacent the interior surface thereof, so that their longitudinal axes are parallel to the rotation axis 31 and their heads protrude above the rim of the container 34. The position of a pair of pinch valve members 70, 71 with respect to a separation bag 1 and the tubes 4, 17, 18 connected thereto when the separation bag 1 rests in the separation cell 40 associated with this pair of pinch valve members 70, 71 is shown in doted lines in figures 1 and 2. Electric power is supplied to the pinch valve members 70, 71 through a slip ring array 38 that is mounted around a lower portion of the rotor shaft 32.

The separation apparatus further comprises four pairs of sensors 73, 74 for monitoring the separation of the various components occurring within each separation bag when the apparatus operates. Each pair of sensors 73, 74 is embedded in the lid 47 of the container 41 of each separation cell 40 along the median longitudinal axis 42 of the container 41, a first sensor 73 being located the farthest and a second sensor 74 being located the closest to the rotation axis 31. When a separation bag 1 rests in the container 41 and the lid 47 is closed, the first sensor 73 (later the bag sensor) faces the upper triangular part of the separation bag 1 and the second sensor 74 (later the tube sensor) faces the proximal end of the first tube 4. The bag sensor 73 is able to detect blood cells in a liquid. The tube sensor 74 is able to detect the presence of absence of liquid in the tube 4 as well as to detect blood cells in a liquid. Each sensor 73, 74 may comprise a photocell including an infrared LED and a photo-detector. Electric power is supplied to the sensors 73, 74 through the slip ring array 38 that is mounted around the lower portion of the rotor shaft 32.

The separation apparatus further comprises a first balancing means for initially balancing the rotor when the weights of the four separation bags 1 contained in the separation cells 40 are different. The first balancing means substantially comprises the same structural elements as the elements of the component transferring means described above, namely: four expandable hydraulic chambers 51 interconnected by a peripheral circular manifold 52, and a hydraulic liquid pumping station 60 for pumping hydraulic liquid into the hydraulic chambers 51 through a rotor duct 56, which is connected to the circular manifold 52. In order to initially balance the rotor, whose four separation cells 40 contain four discrete volumes of a composite liquid that may not have the same weight (because the four volumes may be not equal, and/or the density of the liquid may slightly differ from one volume to the other one), the pumping station 60 is controlled so as to pump into the interconnected hydraulic chambers 51, at the onset of a separation process, a predetermined volume of hydraulic liquid that is so selected as to balance the rotor in the most unbalanced situation. For whole blood, the determination of this balancing volume takes into account the maximum difference in volume between two blood donations, and the maximum difference in hematocrit (i.e. in density) between two blood donations. Under centrifugation forces, the hydraulic liquid will distribute unevenly in the four separation cells 40 depending on the difference in weight of the separation bags 1, and balance the rotor. In order to get an optimal initial balancing, the volume of the cavity 43 of the separation cells 40 should be selected so that the cavities 43, whatever the volume of the separation bags 1 contained therein, are not full after the determined amount of hydraulic liquid has been pumped into the interconnected expansion chambers 51.

The separation apparatus further comprises a second balancing means, for balancing the rotor when the weights of the components transferred into the satellite bags 2, 3, 15 in the central container 34 are different. For example, when two blood donations have the same hematocrit and different volumes, the volumes of plasma extracted from each donation are different, and the same is true when two blood donations have the same volume and different hematocrit. As shown in figures 4, 5, 6 the second balancing means comprises four flexible rectangular pouches 81, 82, 83, 84 that are interconnected by four tube sections 85, 86, 87, 88, each tube section connecting two adjacent pouches by the bottom thereof. The pouches 81, 82, 83, 84 contain a volume of balancing liquid having a density close to the density of the composite liquid. The volume of balancing liquid is so selected as to balance the rotor in the most unbalanced situation. The four pouches 81, 82, 83, 84 are so dimensioned as to line the inner surface of the central container 34 and to have an internal volume that is larger than the volume of balancing liquid so that the balancing liquid can freely expand in any of the pouches 81, 82, 83, 84. In operation, if, for example, four satellite bags 2 respectively adjacent to the four pouches 81, 82, 83, 84 receive different volumes of a plasma component, the four satellite bags 2 will press unevenly, under centrifugation forces, against the four pouches 81, 82, 83, 84, which will result in the balancing liquid becoming unevenly distributed in the four pouches 81, 82, 83, 84 and compensating for the difference in weight in the satellite bags 2.

The separation apparatus further comprises a controller 90 including a control unit (e.g. a microprocessor) and a memory unit for providing the microprocessor with information and programmed instructions relative to various separation protocols (e.g. a protocol for the separation of a plasma component and a blood cell component, or a protocol for the separation of a plasma component, a platelet component, and a red blood cell component) and to the operation of the apparatus in accordance with such separation protocols. In particular, the microprocessor is programmed for receiving information relative to the centrifugation speed(s) at which the rotor is to be rotated during the various stages of a separation process (e.g. stage of component separation, stage of a plasma component expression, stage of suspension of platelets in a plasma fraction, stage of a platelet component expression, etc), and information relative to the various transfer flow rates at which separated components are to be transferred from the separation bag 1 into the satellite bags 2, 3, 15. The information relative to the various transfer flow rates can be expressed, for example, as hydraulic liquid flow rates in the hydraulic circuit, or as rotation speeds of the stepper motor 64 of the hydraulic pumping station 60. The microprocessor is further programmed for receiving, directly or through the memory, information from the pressure gauge 68 and from the four pairs of photocells 73, 74 and for controlling the centrifuge motor 36, the stepper motor 64 of the pumping station 60, and the four pairs of pinch valve members 70, 71 so as to cause the separation apparatus to operate along a selected separation protocol.

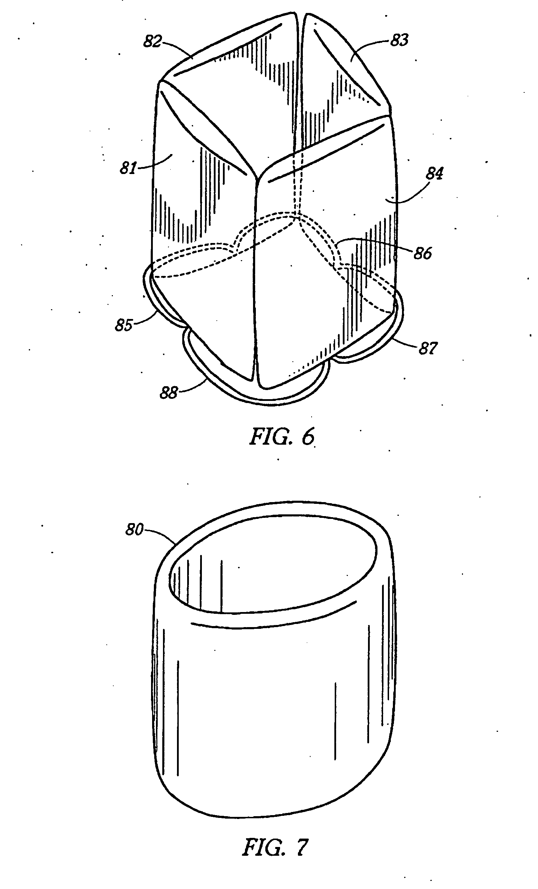

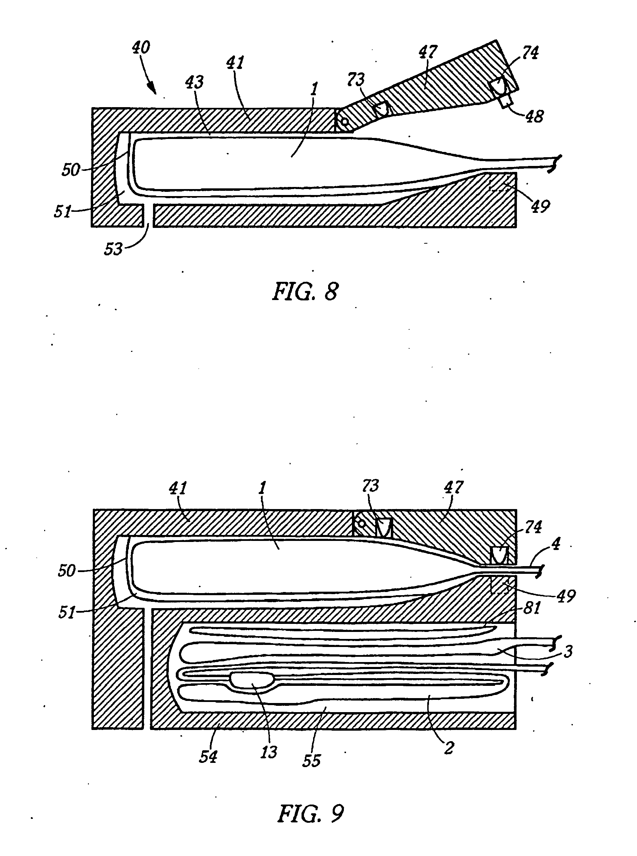

Variants of the first example of the separation apparatus described above are as follows:
- Instead of the centralized hydraulic squeezing system described above, a separation apparatus can be fitted with as many independent squeezing means as separation cells 40. An independent squeezing means may be comprised, for example, of a plate that can be moved by any electro-magnetic, electromechanical or hydraulic mechanism so as to squeeze a separation bag against a wall of the cavity 43 of the container 41 of a separation cell 40.
- Instead of a system of interconnected hydraulic chambers or pouches, the first and/or second balancing means can comprise a ball balancer including a circular cage in which heavy balls can move freely. The circular cage is mounted on the rotor so as to be centered on the rotation axis 31.
- Instead of a central container 34 for containing all the satellite bags 2, 3, 15 connected to the separation bags 1, a separation apparatus can comprise as many satellite bag containers as separation cells. Figure 9 shows a container arrangement that can be used in such a separation apparatus. The container arrangement of figure 9 comprises a separation bag container 41 that is connected to or is made integral with a satellite bag container 54. The satellite bag container 54 comprises a cavity 55 having the shape of a rectangular parallelepiped, which contains a pouch 81 of a balancing assembly as shown in figure 6. The separation bag container 41 is superimposed on the satellite bag container 54 so that the openings of both containers are in the same plane, facing the rotation axis 31 when the container arrangement is mounted on a rotor turntable 35.
- The second sensors 74 can be embedded in the lids 47 of the containers 41 so as to face an upper part of a separation bag 1 close to the connection thereof to the first tube 4.
- The diaphragm 50, instead of being secured to the container 41 so as to line a portion of the lower wall of the cavity 43, can be secured to the container 41 so as to line a portion of the upper wall of the cavity 43.
- In each separation cell 40, the hydraulic chamber 51, instead of being defined by a flexible diaphragm 50 lining the bottom wall of the cavity 43 and a large portion of the lower wall of the cavity 43, can comprise a flexible pouch similar to a pouch of the second balancing means.
- The second balancing means, instead of comprising four interconnected pouches 81, 82, 83, 84 as shown in figure 6, can comprise a flexible tubular pouch 80 having two concentric walls as shown in figure 7. The pouch 80 is so dimensioned as to line the inner surface of the central container 34 and to have an Internal volume that is larger than the volume of balancing liquid so that the balancing liquid can freely expand in one area of pouch or in another.
- The pumping station 60, instead of a piston pump 61, 62, can comprise any pump (e.g. a positive displacement pump) whose output can be controlled with sufficient accuracy.

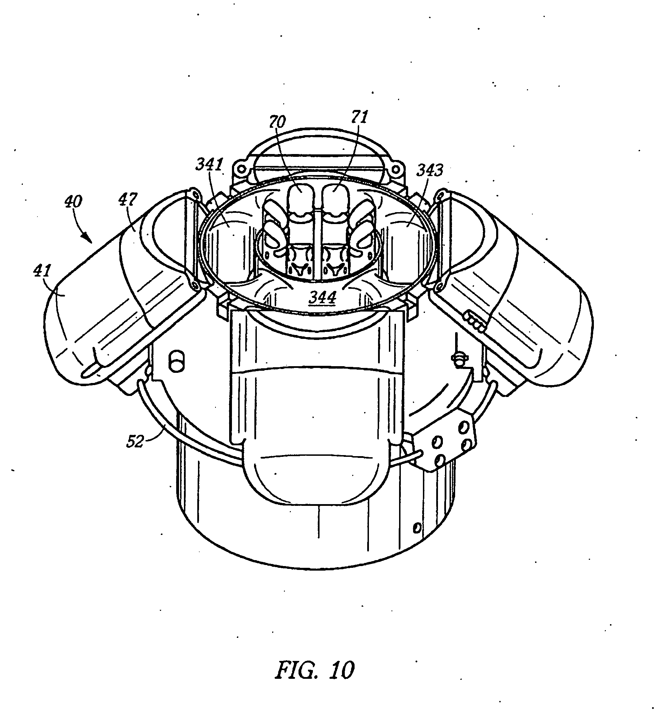

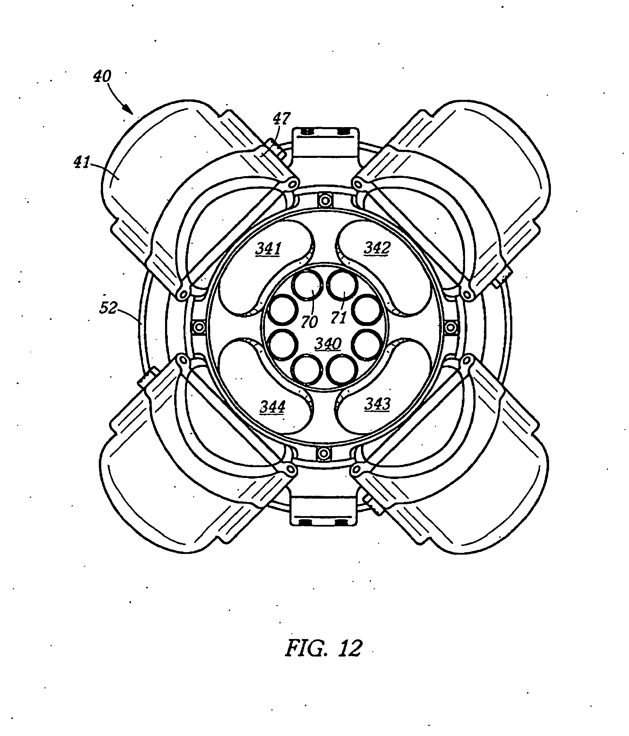

Figures 10, 11, 12 show the rotor of a second example of a separation apparatus for four discrete volumes of a composite liquid.

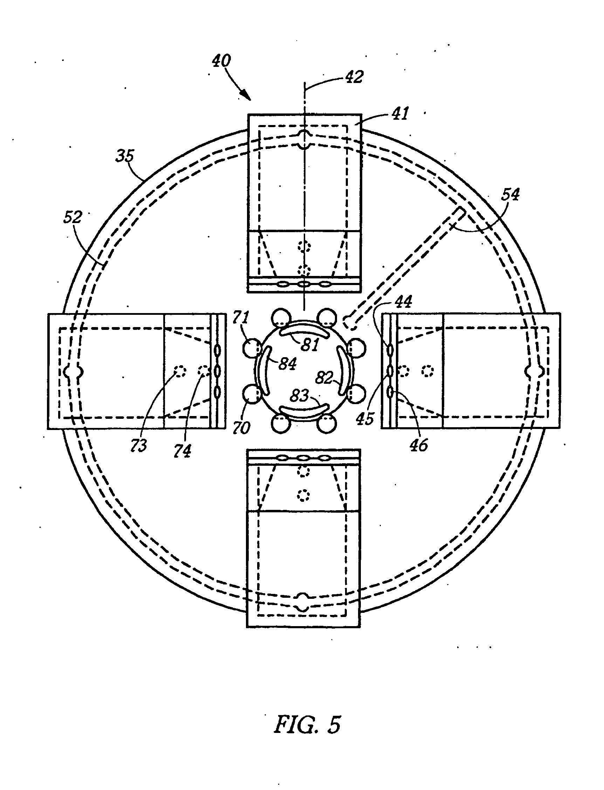

The rotor of this second example essentially differs from the rotor of the embodiment of figures 4 and 5 in the spatial arrangement of the pinch valve members 70, 71 and of the storage means for the satellite bags with respect to the separation cells 40. In this example, the storage means, instead of comprising a central container, comprises four satellite containers 341, 342, 343, 344 that are arranged around a central cylindrical cavity 340, in which the four pairs of pinch valve member 70, 71 are mounted with their longitudinal axes parallel to the rotation axis 31. The cavity 43 of a satellite container 341, 342, 343, 344 has a regular bean-like cross-section, and a central longitudinal axis that is parallel to the rotation axis 31 and intersects the longitudinal axis 42 of the associated separation cell 40.

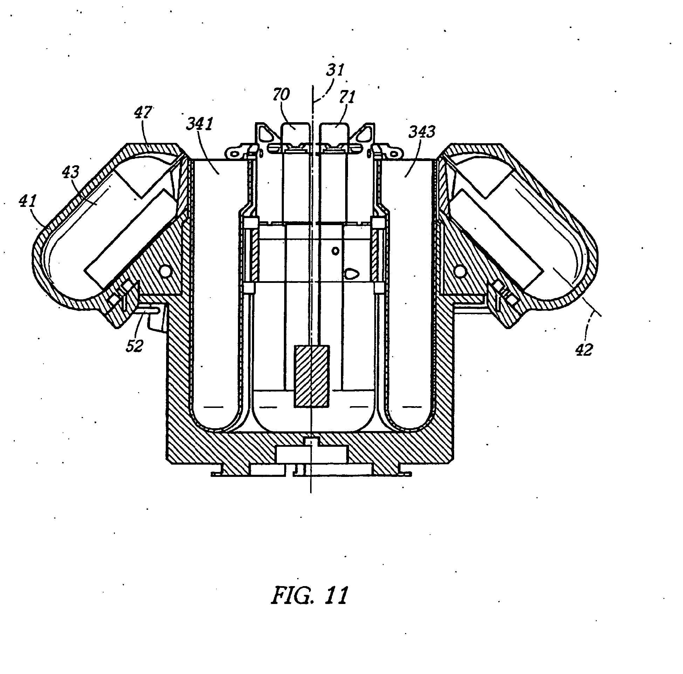

When a set of bag as shown in figures 2, 3a, 3b is mounted on the rotor of figures 11 to 12, the separation bag 1 and the satellite bags 2, 3, 15 are located beyond the associated pinch valves members 70, 71 with respect to the rotation axis 31. The tubes 4, 17, 18 and the three-way connector 16, 16a, 16b connecting the bags are then in the position shown in figures 2, 3a, 3b.

The operation of the separation apparatus of figures 3 and 4, in accordance to a first and second an illustrative separation protocols, will be described now.

According to a **first separation protocol**, four discrete volumes of blood are separated into a plasma component, a first cell component comprising platelets, white blood cells, some red blood cells and a small volume of plasma (later the "buffy coat" component) and a second cell component mainly comprising red blood cells. Each volume of blood is contained in a separation bag 1 of a bag set represented in figure 2, in which it has previously been collected from a donor using the collection tube 5. After the blood collection, the collection tube 5 has been sealed and cut close to the separation bag. Typically, the volumes of blood are not the same in the four separation bags 1, and the hematocrit varies from one separation bag 1 to another one. Consequently, the separation bags 1 have slightly different weights.

### First stage (first protocol): setting the four bag sets in the separation apparatus

Four separation bags 1 are loaded into the four separation cells 40. The lids 47 are closed and locked, whereby the separation bags 1 are secured by their upper edge to the containers 41 (the pins 48 of the securing means pass then through the holes 8 in the upper corner of the separation bags 1 and engage the recesses 49 or the securing means).

The tubes 17 connecting the separations bags 1 to the plasma component bags 2, through the T connectors 16, are inserted in the groove 72 of the first pinch valve members 70. The tubes 18 connecting the separations bags 1 to the buffy coat component bags 15, through the T connector 16, are inserted in the groove 72 of the second pinch valve members 71. The four plasma component bags 2, the four buffy coat component bags 15, the four red blood cell component bags 3 and the four leuko-reduction filters 13 are inserted in the central compartment 34 of the rotor. The four plasma component bags 2 are respectively placed in direct contact with the pouches 81 to 84 of the second balancing means. The pinch valve members 70, 71 are closed and the breakable stoppers 9 in the tubes 4 connecting the separation bags 1 to the T connectors 16 are manually broken.

### Second stage (first protocol): balancing the rotor in order to compensate for the difference in weights of the separation bags

At the onset of the second stage, all the pinch valve members 70, 71 are closed. The rotor is set in motion by the centrifuge motor 36 and its rotation speed increases steadily until it rotates at a first centrifugation speed. The pumping station 60 is actuated so as to pump a predetermined overall volume of hydraulic liquid into the four hydraulic chambers 51, at a constant flow rate. This overall volume of liquid is predetermined taking into account the maximum variation of weight between blood donations, so that, at the end of the second stage, the weights in the various separation cells 40 are substantially equal and the rotor is substantially balanced, whatever the specific weights of the separation bags 1 that are loaded in the separation cells 40. Note that this does not imply that the internal cavity 43 of the separation cells 40 should be filled up at the end of the balancing stage. For the purpose of balancing the rotor, it suffices that there is enough hydraulic liquid in the separation cells 40 for equalizing the weights therein, and it does not matter if an empty space remains in each separation cell 40 (the size of this empty space essentially depends on the volume of the internal cavity 43 of a separation cell 40 and the average volume of a blood donation). Because the hydraulic chambers 51 are interconnected, the distribution of the overall volume of hydraulic liquid between the separations chambers 40 simply results from the rotation of the rotor. When the weights of the separation bags 1 are the same, the distribution of the hydraulic liquid is even. When they are not, the distribution of the hydraulic liquid is uneven, and the smaller the weight of a specific separation bag 1, the larger the volume of the hydraulic fluid in the associated hydraulic chamber 51.

### Third stage (first protocol): the blood within the separation bags 1 is sedimented to a desired level.

At the onset of this stage, all pinch valve members 70, 71 are closed. The rotor is rotated at a second centrifugation speed (high sedimentation speed or "hard spin") for a predetermined period of time that is so selected that, whatever the hematocrit of the blood in the separation bags 1, the blood sediments in each of the separation bag 1 at the end of the selected period to a point where the hematocrit of the outer red blood cell layer is about 90 and the inner plasma layer does not substantially contain anymore cells, the platelets and the white blood cells forming then an intermediary layer between the red blood cell layer and the plasma layer.

### Fourth stage (first protocol): a plasma component is transferred into the plasma component bags 2.

At the onset of this stage, the rotation speed is decreased to a third centrifugation speed, the four first pinch valve members 70 controlling access to the plasma component bags 2 are opened, and the pumping station 60 is actuated so as to pump hydraulic liquid at a first constant flow rate into the hydraulic chambers 51 and consequently squeeze the separation bags 1 and cause the transfer of plasma into the plasma component bags 2.

When blood cells are detected by the bag sensor 73 in the separation cell 40 in which this detection occurs first, the pumping station 60 is stopped and the corresponding first pinch valve member 70 is closed, either immediately of after a predetermined amount of time selected in view of the volume of plasma that it is desirable in the buffy coat component to be expressed in a next stage.

Following the closure of the first (first) pinch valve member 70 (i.e. the first pinch valve of the group of first pinch valve members 70) to close, the pumping station 60 is actuated anew so as to pump hydraulic liquid at a second, lower, flow rate into the hydraulic chambers 51 and consequently squeeze the three separation bags 1 whose outlet is not closed by the corresponding first pinch valve members 70.

When blood cells are detected by the bag sensor 73 in the separation cell 40 in which this detection occurs second, the pumping station 60 is stopped and the corresponding first pinch valve member 70 is closed (same timing as for the closing of the first (first) pinch valve member to close).

Following the closure of the second (first) pinch valve member 70 to close, the pumping station 60 is actuated anew so as to pump hydraulic liquid at the second flow rate into the hydraulic chambers 51 and consequently squeeze the two separation bags 1 whose outlet is not closed by the corresponding first pinch valve members 70.

When blood cells are detected by the bag sensor 73 in the separation cell 40 in which this detection occurs third, the pumping station 60 is stopped and the corresponding first pinch valve member 70 is closed (same timing as for the dosing of the first (first) pinch valve member to close).

Following the closure of the third (first) pinch valve member 70 to close, the pumping station 60 is actuated anew so as to pump hydraulic liquid at the second flow rate into the hydraulic chambers 51 and consequently squeeze the separation bag 1 whose outlet is not yet closed by the corresponding first pinch valve member 70.

When blood cells are detected by the bag sensor 73 in the separation cell 40 in which this detection occurs last, the pumping station 60 is stopped and the corresponding first pinch valve member 70 is closed (same timing as for the closing of the first pinch valve member to close).

In the plasma component transfer process described above, the transfer of the four plasma components starts at the same time, run in part simultaneously and stop independently of each other upon the occurrence of a specific event in each separation bag (detection of blood cells by the bag sensor).

As a variant, when the second flow rate is sufficiently low and the closing of the first pinch valve member 70 occurs almost simultaneously with the detection of blood cells in the separation bags, then the pumping station can be continuously actuated during the fourth stage.

The fourth stage ends when the four first pinch valve members 70 are closed.

### Fifth stage (first protocol): a buffy coat component is transferred into the buffy coat component bags 15.

The control unit 90 is programmed to start the fifth stage after the four first pinch valve members 70 are closed, upon receiving information from the last bag sensor 73 to detect blood cells.

At the onset of this stage, the rotation speed remains the same (third centrifugation speed), a first of the four second pinch valve members 71 controlling access to the buffy coat component bags 15 is opened, and the pumping station 60 is actuated so as to pump hydraulic liquid at a third constant flow rate into the hydraulic chambers 51 and consequently squeeze the separation bag 1 in the separation cell 40 associated with the opened second pinch valve members 71 and cause the transfer of the buffy coat component into the buffy coat component bag 2 connected to this separation bag 1.

After a predetermined period of time after blood cells are detected by the tube sensor 74 in the separation cell 40 associated with the opened second pinch valve member 71, the pumping station 60 is stopped and the second pinch valve member 71 is closed.

After the first (second) pinch valve member 71 has closed (i.e. the first pinch valve of the group of second pinch valve members 71), a second (second) pinch valve member 71 is opened, and a second buffy coat component is transferred into a buffy coat component bag 2, in the same way as above.

The same process is successively carried out to transfer the buffy coat component from the two remaining separation bags 1 into the buffy coat component bag 2 connected thereto.

In the buffy coat component transfer process described above, the transfers of the four buffy coat components are successive, and the order of succession is predetermined. However, each of the second, third and four transfers starts following the occurrence of a specific event at the end of the previous transfer (detection of blood cells by the tube sensor 74 or closing of the second valve member 71).

As a variant, when the third flow rate is sufficiently low and the closing of the second pinch valve members 71 occurs almost simultaneously with the detection of blood cells in the tubes 4, then the pumping station can be actuated continuously during the fourth stage.

As a variant, the control unit 90 is programmed to start the fifth stage after a predetermined period of time after receiving information from the first (or the second or the third) bag sensor 73 to detect blood cells. The period of time is statistically or empirically determined so that, whatever the event from which it starts running (detection of the blood cells by either one of the first, second, and third bag sensor 73 to detect blood cells), the four first pinch valve members 70 are closed when it is over.

The fifth stage ends when the four second pinch valve members 71 are closed.

### Sixth stage (first protocol): the centrifugation process is ended.

The control unit 90 is programmed to start the sixth stage after the four (second) pinch valve members 71 are dosed, upon receiving information from the last tube sensor 74 to detect blood cells.

The rotation speed of the rotor is decreased until the rotor stops, the pumping station 60 is actuated so as to pump the hydraulic liquid from the hydraulic chambers 51 at a high flow rate until the hydraulic chambers 51 are empty, and the first and second pinch valve members 70, 71 are actuated so as to seal and cut the tubes 17, 18. The blood cells remain in the separation bags 1.

When the fifth stage is completed, the four bag sets are removed from the separation apparatus and each bag set is separately handled manually.

The breakable stopper 10 blocking the communication between the separation bag 1 and the tube 6 connected thereto is broken, as well as the breakable stopper 14 blocking the communication between the second satellite bag 3 and the tube 6. The storage solution contained in the second satellite bag 3 is allowed to flow by gravity through the leuko-reduction filter 13 and into the separation bag 1, where it is mixed with the red blood cells so as to lower the viscosity thereof. The content of the separation bag 1 is then allowed to flow by gravity through the filter 13 and into the second satellite bag 3. The white blood cells are trapped by the filter 13, so that substantially only red blood cells are collected into the second satellite bag 3.

As a variant, the control unit 90 is programmed to start the sixth stage after a predetermined period of time after receiving information from the first (or the second or the third) tube sensor 74 to detect blood cells. The period of time is statistically or empirically determined so that, whatever the event from which it starts running (detection of the blood cells by either one of the first, second, and third tube sensor 74 to detect blood cells), the four second pinch valve members 71 are closed when it is over.

According to a **second separation protocol**, four discrete volumes of blood are separated into a plasma component, a platelet component and a red blood cell component Each volume of blood is contained in a separation bag 1 of a bag set represented in figure 2, in which it has previously been collected from a donor using the collection tube 5. After the blood collection, the collection tube 5 has been sealed and cut close to the separation bag 1. Typically, the volumes of blood are not the same in the four separation bags 1, which, consequently, have slightly different weights. Also, typically, the hematocrit varies from one separation bag 1 to another one.

### First stage (second protocol): setting the four bag sets in the separation apparatus

This stage is identical to the first stage of the first protocol.

### Second stage (second protocol): balancing the rotor in order to compensate for the difference in weights of the separation bags

This stage is identical to the second stage of the first protocol.

### Third stage (second protocol): the blood within the separation bags 1 is sedimented to a desired level.

This stage is identical to the third stage of the first protocol.

### Fourth stage (second protocol): a first, larger, portion of plasma is transferred into the plasma bags 2, while a second, smaller, portion of plasma remains in the separation bags 1.

This stage is substantially the same as the fourth stage of the first protocol. However, the expression of plasma from each separation bag 1 into the attached plasma component bag 2 is stopped immediately after detection of blood cells by the corresponding bag sensor 73, so that the volume of plasma remaining in the separation bag 1 is large enough to allow the platelets to be re-suspended therein.

### Fifth stage (second protocol): a platelet component is prepared in the separation bag 1.

At the onset of this fifth stage, the first and second valve members 70, 71 are closed. The rotor is stopped and the pumping station 60 is actuated so as to pump a volume of hydraulic liquid from the hydraulic chambers 51 at a high flow rate. The rotor is then controlled so as to oscillate back and forth around the rotation axis 31 for a determined period of time, at the end of which the cells in the separation bags 1 are substantially suspended in plasma. The rotor is then set in motion again by the centrifuge motor 36 so that its rotation speed increases steadily until it reaches a fourth centrifugation speed (low sedimentation speed or "soft spin"). The rotor is rotated at the fourth rotation speed for a predetermined period of time that is selected so that the blood sediments in the separation bags 1 at the end of the selected period to a point where the separation bags 1 exhibit an outer layer comprising packed red blood cells and an inner annular layer substantially comprising platelets suspended in plasma.

### Sixth stage (second protocol): a platelet component is transferred into the platelet bags 15.

This stage is substantially the same as the fifth stage of the first protocol (buffy coat expression).

### Seventh stage (second protocol): the centrifugation process is ended.

This stage is substantially the same as the sixth stage of the first protocol.

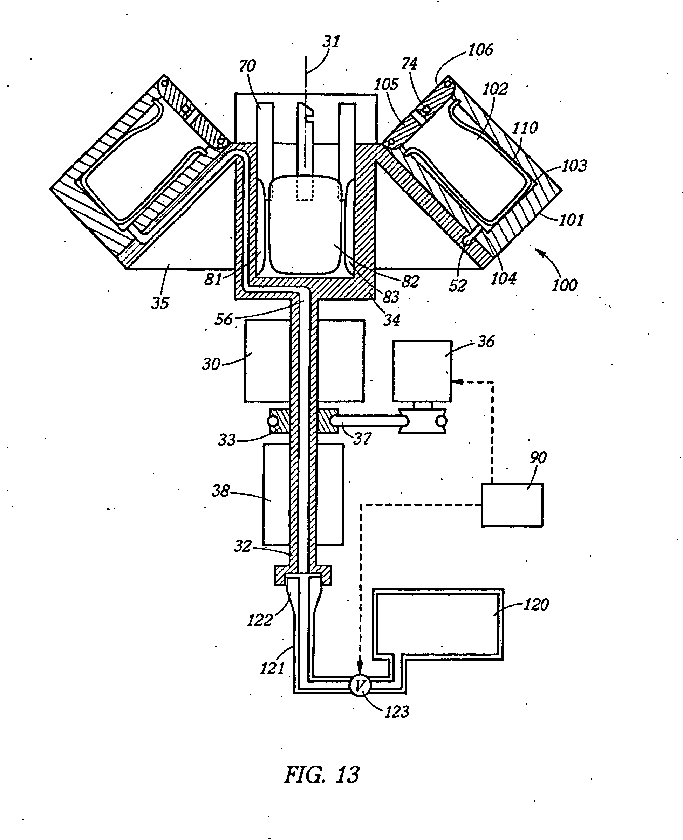

Figures 13 to 18 show a third example of a separation apparatus for four discrete volumes of a composite liquid.

The separation apparatus of figure 13 to 18 is particularly adapted to the separation of a composite fluid in two components, for example the separation of whole blood into a cell component (red blood cells, white cells and platelets) and a plasma component substantially devoid of cells or the separation of whole blood into a cell component (red blood cells, white cells and a small amount of platelets) and a plasma component containing a large amount of platelets in suspension.

The main differences between the first separation apparatus shown in figures 4 and 5 and the third separation apparatus shown in figures 13 to 18 are as follows:
- The shape of the separation cells 100 of the third separation apparatus is different from the shape of the separation cells 40 of the first separation apparatus.
- Each of the separation cells 100 of the third separation apparatus is associated with one pinch valve member 70 and one tube sensor 74.
- The third separation apparatus does not comprise a pumping station for pumping a hydraulic liquid in and out of the hydraulic chambers of the separation cells 100.

In more details, a separation cell 100 for the third separation apparatus comprises a container 101 having the general shape of a rectangular parallelepiped. The cavity (also referred to as the "separation compartment") of the container 101, which has also the general shape of a rectangular parallelepiped, is so dimensioned as to loosely accommodate a separation bag 1 full of liquid, of the type shown in figure 2. The separation cell 100 further comprises an elastic diaphragm 110, which defines within the cavity of the container 101 a first chamber 102 for receiving a separation bag 1, and a second hydraulic chamber 103 that is connected to the peripheral manifold 52, through an inlet aperture 104 close to the bottom of the container 101. The separation cell 100 further comprises a lid having two flaps 105, 106 that are hinged to the longer parallel sides of the opening of the container 101. The two flaps 105, 106 can be locked in a closed position by a locking means (not shown). The separation cell 100 further comprises a securing means for securing a separation bag 1 within the separation cell 100. The bag securing means comprises two pins 107 and two corresponding recesses 108 that respectively protrude or open on the edges of the flaps 105, 106 that face each other when the lid is closed. The two pins 107 are so spaced apart and dimensioned as to fit into the two holes 8 in the upper corner of a separation bag 1. The two flaps 105, 106 also comprise on their facing edges three semi-cylindrical holes 109 for accommodating the proximal end of three tubes 4, 5, 6 embedded in the upper area of a separation bag 1. The outer flap 106 includes a cavity facing the median semi-cylindrical hole 109, for containing the bag sensor 74.

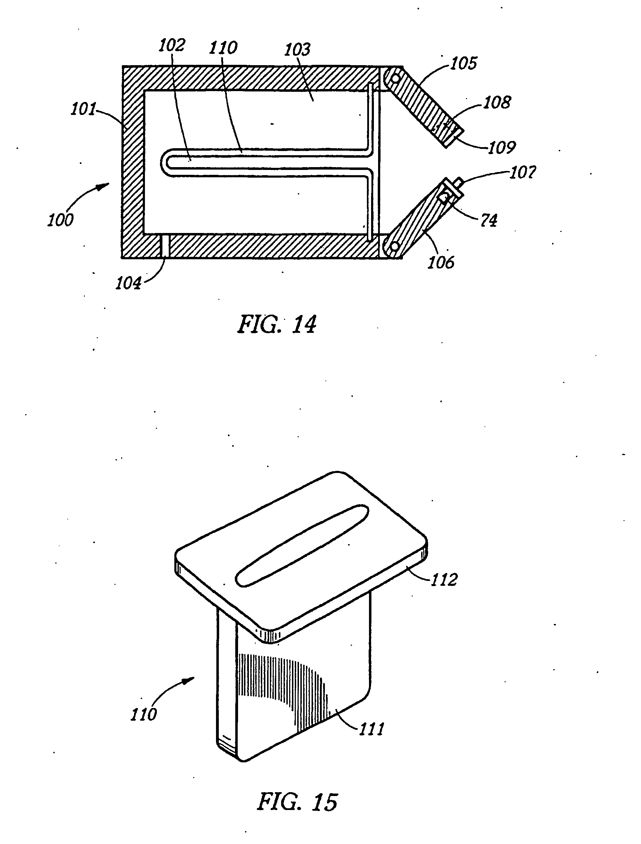

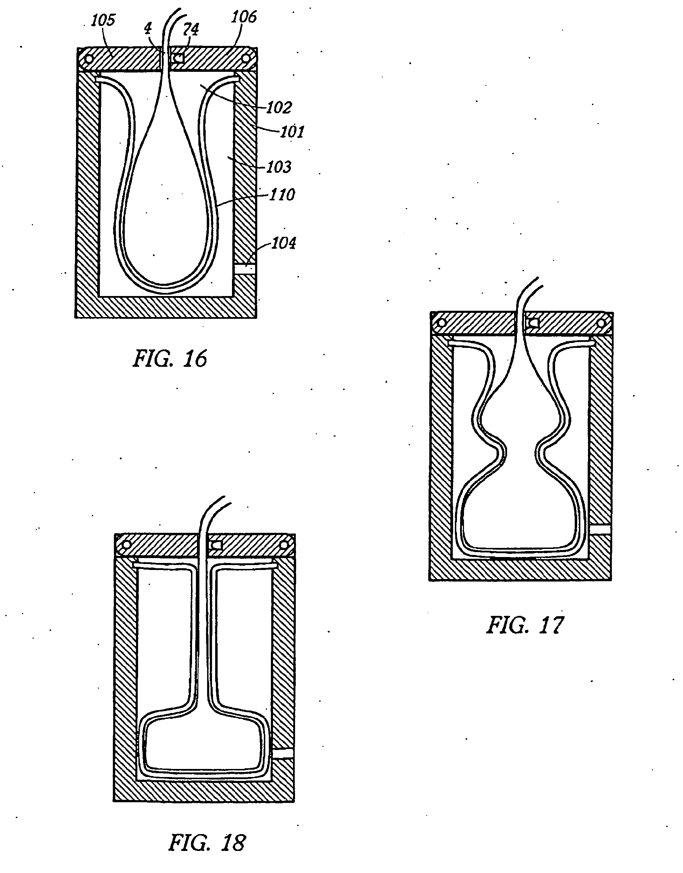

As shown in figures 15 to 18, the diaphragm 110 comprises a flat rectangular socket 111 almost as wide as a separation cell 100. The diaphragm 110 further comprises a large, rectangular, connecting portion 112 extending around the mouth of the socket 111, perpendicularly to the socket 111 when the diaphragm 110 is not deformed by a separation bag 1 and it is held in an upright position (figure 15). The socket 111 is connected to the connecting portion 112 along the longitudinal median axis thereof. The connecting portion 112 has a surface slightly larger than a transversal cross-section of the cavity of the container 101. The diaphragm 110 is tightly attached to the top of the container 101 by a peripheral area of the connecting portion 112. The diaphragm 110 is made of an elastic and deformable elastomeric material so selected that the diaphragm 110 conforms very closely the shape of a separation bag 1 before and during centrifugation and as shown in figures 16 to 18.

As mentioned above, the separation apparatus shown in figure 13 does not comprise a pumping station for pumping a hydraulic fluid in and out of the hydraulic chambers 103. Instead, it comprises a reservoir 120 for hydraulic liquid, which is fixed with respect to the rotor, and which is directly connected to the rotor duct 56 by a conduit 121 and a rotary seal 122. The conduit 121 is fitted with a valve 123. The reservoir 120 is secured to a frame of the separation apparatus so as to be lower than the four separation cells 100. When the separation apparatus is used for separating red blood cells from plasma (with or without suspended platelets), the density of the hydraulic liquid is selected, for reasons explained below, so as to be between the density of packed red blood cells and the density of plasma.

The component transferring means of the third separation apparatus essentially comprises the reservoir 120 that is directly connected to the rotor duct 56 by the rotary seal 122, the hydraulic chambers 103, and the motor 36 that drives the rotor in rotation. When the valve 123 is opened and the rotation speed of the rotor reaches a determined threshold, which depends on the height between the reservoir 120 and the separation cells 100 and the distance between the rotation axis 31 and the separation cells 100, then the hydraulic liquid flows from the reservoir 120 into the hydraulic chambers 103 so as to fill up the hydraulic chamber 103 and squeeze the separation bags 1 therein, whatever the volume/weight of the separation bags 1. The speed threshold is substantially below the rotation speed at which the rotor is rotated for separating blood components ("high spin" as well as "soft spin). The transfer of a separated component from a separation bag 1 into a satellite bag 2 is then controlled by the opening/closing of the pinch valve member 70 in which the tube 4 connecting the two bags is inserted.

The first balancing means of the third separation apparatus essentially comprises the reservoir 120 that is directly connected to the rotor duct 56 through the rotary seal 122, the hydraulic chambers 103, the motor 36 that drives the rotor in rotation, and the valve 123. At the onset of a separation process, the valve 123 is opened for a predetermined period of time so as to allow the transfer, in the interconnected hydraulic chambers 103, of a predetermined volume of hydraulic liquid that is so selected as to balance the rotor in the most unbalanced situation. For whole blood, the determination of this balancing volume takes into account the maximum difference in volume between two blood donations, and the maximum difference in hematocrit (i.e. in density) between two blood donations.

A variant of the third example of a separation apparatus does not comprise a valve 123 on the conduit 121 connecting the reservoir 120 to the rotor duct 56. As a result, when the threshold speed is reached, the hydraulic liquid is pumped from the reservoir 120 into the hydraulic chambers 103 until the pressure that is building up within the separation cells 100 prevents further pumping. The filling up of the space available in the separation cells 100 with hydraulic liquid might not however result in an optimal balance of the rotor depending, in particular, on the difference in weight of the separation bags 1, of their volume, and of the density of the hydraulic liquid.

The operation of the third separation apparatus, in accordance to a third illustrative separation protocol, will be described now.

According to a **third separation protocol**, four discrete volumes of blood are separated into a plasma component (including or not including a substantial amount of platelets) and a blood cell component (including platelets, or residual platelets, white blood cells and red blood cells). Each volume of blood is contained in a separation bag 1 of a bag set represented in figure 1, in which it has previously been collected from a donor using the collection tube 5. After the blood collection, the collection tube 5 has been sealed and cut close to the separation bag 1. Typically, the volumes of blood are not the same in the four separation bags 1 and the hematocrit varies from one separation bag 1 to another one. As a result, the separation bags have slightly different weights.

### First stage (third protocol): setting the four bag sets in the separation apparatus

Four separation bags 1 are inserted into the socket 111 of a diaphragm 110 within the four separation cells 100 as shown in figure 16. The two flaps 105, 106 of the lids of the separation cells 100 are closed and consequently secure the top of the separation bags 1 to the separation cells 100. The tube sensors 74 embedded in the outer flap 106 of the lids now face the proximal end of the tubes 4 connecting the separation bags 1 to the plasma component bags 2. The tubes 4 are inserted in the groove 72 of the pinch valve members 70. The four plasma component bags 2, the four red blood cell component bags 3 and the four leuko-reduction filters 13 are inserted in the central compartment 34 of the rotor. The pinch valve members 70 are closed and the breakable stoppers 9 in the tubes 4 connected to the plasma component bags 2 are manually broken.

### Second stage (third protocol): balancing the rotor in order to compensate for the difference in weights of the separation bags

At the onset of this second stage, the pinch valve members 70, in which the tubes 4 are engaged, are closed. The valve 123 on the conduit connecting the reservoir 120 to the rotor duct 56 is opened. The rotor is set in motion by the centrifuge motor 36 and its rotation speed increases steadily until it rotates at a predetermined sedimentation speed. Before it rotates at the sedimentation speed, the rotor reaches a threshold speed at which its rotation causes the pumping of hydraulic liquid from the reservoir 120 into the interconnected hydraulic chambers 103 of the separation cells 100. The valve is closed 123 after a predetermined amount of hydraulic fluid sufficient for balancing the rotor has been transferred in the hydraulic chambers 103. Because the hydraulic chambers 103 are interconnected by the peripheral manifold 52, the hydraulic liquid gets automatically distributed in the separation cells 100 so as to balance the rotor. When the weights of the separation bags 1 are the same, the distribution of the hydraulic liquid is even. When they are not, the distribution of the hydraulic liquid is uneven, and the smaller the weight of blood in a specific separation bag 1, the larger the volume of the hydraulic fluid in the associated hydraulic chamber 103.

### Third stage (third protocol): the blood within the separation bags 1 is sedimented to a desired level.

When it is desired to separate a plasma component containing a large amount of suspended platelets ("platelet rich plasma") and a cell component mainly containing red blood cells and white blood cells, the rotor is rotated at a first sedimentation speed (about 2000 RPM, usually referred to as "soft spin").
When it is desired to separate a plasma component substantially devoid of cells ("platelet poor plasma") and a cell component containing red blood cells, white blood cells and platelets, the rotor is rotated at a second sedimentation speed (about 3200 RPM, usually referred to as "hard spin").
The rotor is rotated at the selected sedimentation speed for a predetermined period of time that is selected so that, whatever the hematocrit of the blood in the separation bags 1, the blood sediments at the desired level in each of the separation bag 1 at the end of the selected period. Since, as mentioned above, the density of the hydraulic liquid is selected so as to be between the density of the packed red cells and the density of the plasma, the separation bag 1 will take a hour-glass shape at the end of the sedimentation stage, as shown in figure 17.

### Fourth stage (third protocol): a plasma component is transferred into the satellite bags 2.

At the onset of this stage, the four pinch valve members 70 controlling the access to the plasma component bags 2 are opened. This causes a decrease in pressure within the separation cells 100 and hydraulic liquid starts flowing again into the hydraulic chambers 103. The raising volume of hydraulic fluid in the hydraulic chamber 103 squeezes the separation bags 1 and causes the transfer of the plasma component into the first satellite bags 2. Because the hydraulic liquid has a lower density than the density of the packed red blood cells, the red blood cells remain at the bottom of the separation cell 100 and the separation bags 1 progressively collapse above the red cells as shown in figure 18.

When each tube sensor 74 detects blood cells, then the associated pinch valve member 70 is closed. When the volumes of blood in the four separation bags 1 are different, and/or the hematocrit of the blood in the four separation bags 1 is different (which will be generally the case), then the four pinch valve members 70 close one after the other.

The fourth stage end when the four pinch valve members 70 are closed.

### Fifth stage (third protocol): the centrifugation process is ended.

When the last pinch valve member 70 closes, the rotation speed of the rotor is decreased until the rotor stops. The hydraulic liquid simultaneously drains from the hydraulic chambers 103 into the reservoir 120. The red blood cells and the white blood cells remain in the separation bag 1 (as well as the platelets when the plasma component collected is a "platelet poor plasma").

When the fifth stage is completed, the four bag sets are removed from the separation apparatus and each bag set is separately handled manually.

The breakable stopper 10 blocking the communication between the separation bag 1 and the tube 6 connected thereto is broken, as well as the breakable stopper 14 blocking the communication between the second satellite bag 3 and the tube 6. The storage solution contained in the second satellite bag 3 is allowed to flow by gravity through the filter 13 and into the separation bag 1, where it is mixed with the blood cells so as to lower the viscosity thereof. The content of the separation bag 1 is then allowed to flow by gravity through the filter 13 and into the second satellite bag 3. The white blood cells and the platelets are trapped by the filter 13, so that substantially only red blood cells are collected into the second satellite bag 3.

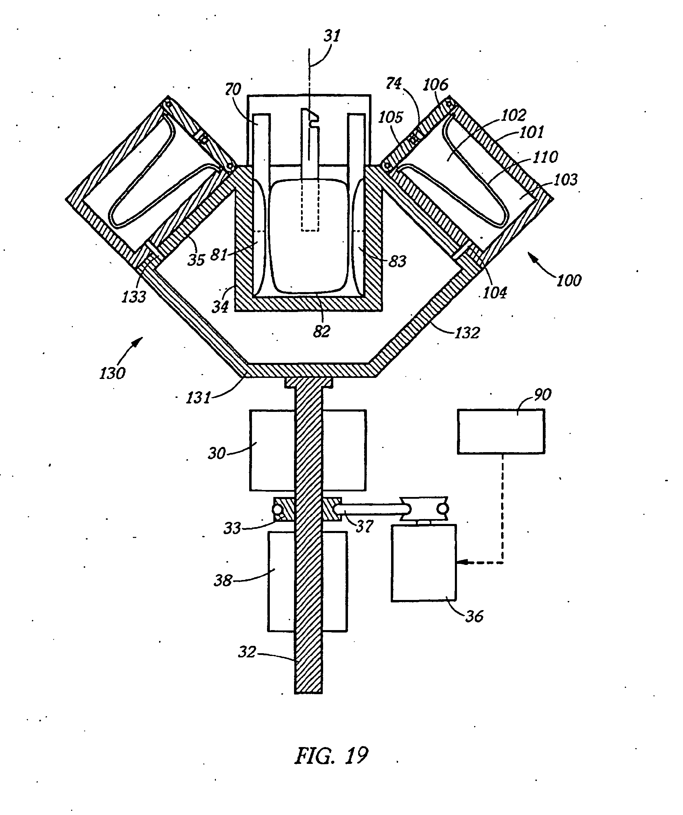

Figure 19 shows a fourth example of a separation apparatus for four discrete volumes of a composite liquid.

The main differences between the third separation apparatus shown in figures 13 to 18 and the fourth separation apparatus shown in figure 19 are as follows:
- The fourth separation apparatus does not comprise a fixed reservoir directly connected to the separation chambers, via a conduit, a rotary seal and a rotor duct;
- The fourth separation apparatus comprises a hydraulic liquid reservoir 130 that is mounted on the rotor.

The rotor of the apparatus of figure 19 comprises:
- A central container 34 for satellite bags, having the shape of a cylindrical bucket;
- A turntable 35 having a frusto-conical wall supporting four separation cells 100 at an angle with respect to the rotation axis 31; the turntable 35 is connected by its smaller diameter section to an upper rim of the central container 34 so as to flare underneath the rim of the central container 34;
- A reservoir 130 for hydraulic liquid, which comprises a circular bottom wall 131 and frusto-conical wall 132 connected by its smaller diameter section to the circular bottom wall 131 and by its larger diameter section to the lower rim of the turntable 35 (i.e. the section of the turntable having the larger diameter). In other words, the interior of the reservoir 130 has a complex geometrical volume that is symmetrical with respect to the rotation axis 31 and that is defined by the outside surface or the central container 34, the inner surface of the turntable 35, the inner surface of the frusto-conical wall 132 of the reservoir, and the inner surface of the bottom wall 131 of the reservoir.
- A rotor shaft 32, which is connected to the bottom wall of the reservoir 130.

The reservoir 130 is fluidly connected to the hydraulic chamber 103 of each separation cell 100 by an outlet aperture 133 through the turntable 35 that coincides with the inlet aperture 104 of the hydraulic chambers 103. As shown, the outlet apertures 133 are located the farthest from the rotation axis 31. With this arrangement, the hydraulic liquid flows from the reservoir 130 into the hydraulic chambers 103 of the separation cells 100 under centrifugal forces as soon as the rotor starts rotating. When the separation apparatus is to be used for separating red blood cells from plasma (with or without suspended platelets), the density of the hydraulic fluid is selected so as to be between the density of pack red cells and the density of plasma.

In this fourth example of a separation apparatus, the component transferring means essentially comprise the reservoir 130, the hydraulic chambers 103 and the motor 36 that drives the rotor in rotation. When the rotor rotates, the hydraulic liquid drains from the reservoir 130 into the hydraulic chambers 103 under centrifugal forces and presses the separation bags 1 within the separation cell 100 through the elastic diaphragm 110. The transfer of a separated component from a separation bag 1 into a satellite bag 2 is controlled by the opening/closing of the pinch valve member 70 in which the tube 4 connecting the two bags is inserted.

The first balancing means essentially comprise the reservoir 130, the hydraulic chambers 103 and the motor 36 that drives the rotor in rotation. As soon as the rotor starts rotating, hydraulic fluid flows from the reservoir 130 into the hydraulic chambers 103 until it completely fills up the space let vacant in the separation cells 100 by the separation bags 1, which happens before the rotor has reach the desired sedimentation speed. The filling up of the space available in the separation cells 100 with hydraulic liquid might not however result in an optimal balance of the rotor depending, in particular, on the difference in weight of the separation bags 1, on their volume, and on the density of the hydraulic liquid.

It will be apparent to those skilled in the art that various modifications can be made to the apparatus and method described herein. Thus, it should be understood that the invention is not limited to the subject matter discussed in the specification. Rather, the present invention is intended to cover modifications and variations.

## Claims

1. A set of bags for the separation of a composite liquid into at least two components in a centrifuge having a rotation axis, comprising:
- a collection and separation bag (1) having a median axis, a top and a bottom, wherein the top of the separation bag comprises two edges converging towards a tip located on the median axis;
- a collection tube (5) having a first end connected to the collection and separation bag (1); and
- at least two satellite bags (2, 3, 15) connected to the top of the collection and separation bag (1);
- a first flexible tube (17) having:
- a first end connected to a first satellite bag (2);
- a valve section for engaging a first pinch valve of the centrifuge;
- a second flexible tube (18) having:
- a first end connected to a second satellite bag (15);
- a valve section for engaging a second pinch valve of the centrifuge; and
- a third tube (4) having:
- a first end connected to the tip of the collection and separation bag (1);
- a second end;
- a three-way connector (16, 16a, 16b) having:
an inlet channel (20) connected to the second end of said third tube (4);
a first outlet channel (21) connected to a second end of the first tube (17), and
a second outlet channel (22) connected to a second end of the second tube (18),
**characterised in that** the first outlet channel (21) and the second outlet channel (22) are perpendicularly connected to the inlet channel (20) and are offset along the inlet channel (20) so that the first outlet channel (21) is further than the second outlet channel (22) from the end of the inlet channel (20) that is connected to the third tube (4).

2. A bag set according to claim 1, wherein the first outlet channel (21) connects to the inlet channel (20) at a first location and the second outlet channel (22) connects to the inlet channel (20) at a second location, the first location being closer to the rotation axis than the second location when the bag set is mounted in the centrifuge with the first valve section engaged in the first pinch valve and the second valve section engaged in the second pinch valve.

3. A bag set according to claim 1 or 2, wherein the third tube (4) comprises a detection section for engaging a cell detector of the centrifuge.

4. A bag set according to claim 1, 2 or 3, further comprising a breakable stopper (9) connected to the third tube (4).

5. A bag set according to any one of claims 1 to 4, wherein the composite liquid is whole blood, said bag set being for the separation of whole blood into a plasma component, a platelet component and a red blood cell component, wherein the first satellite bag (2) is for collecting the plasma component and the second satellite bag (15) is for collecting the platelet component.

6. A bag set according to any one of claims 1 to 5, further comprising:
- a third satellite bag (3);
- a fourth tube (6) having:
- a first section having a first end connected to the top of the collection and separation bag (1); and
- a second section having a second end connected to the third satellite bag (3); and
- a leuko-reduction filter (13) having an inlet connected to a second end of the first section of the fourth tube (6) and an outlet connected to a first end of the second section of the fourth tube (6).

7. A bag set according to any one of the preceding claims, further comprising a needle (12) connected to a second end of the collection tube (5).

## Patentansprüche

1. Beutelset zur Auftrennung einer Flüssigkeit aus mehreren Komponenten in zumindest zwei Komponenten in einer Zentrifuge mit einer Drehachse, das umfasst:
- einen Sammel- und Auftrennbeutel (1) mit einer Mittelachse, einem oberen Bereich und einem unteren Bereich, wobei der obere Bereich des Auftrennbeutels zwei Ränder umfasst, die hin zu einer auf der Mittelachse liegenden Spitze zusammenlaufen;
- ein Sammelrohr (5) mit einem ersten Ende, das mit dem Sammel- und Auftrennbeutel (1) verbunden ist; und
- zumindest zwei Satellitenbeutel (2, 3, 15), die mit dem oberen Bereich des Sammel- und Auftrennbeutels (1) verbunden sind;
- ein erstes flexibles Rohr (17) mit:
- einem ersten Ende, das mit einem ersten Satellitenbeutel (2) verbunden ist;
- einem Ventilabschnitt, um mit einem ersten Quetschventil der Zentrifuge in Eingriff zu gelangen;
- ein zweites flexibles Rohr (18) mit:
- einem ersten Ende, das mit einem zweiten Satellitenbeutel (15) verbunden ist;
- einem Ventilabschnitt, um mit einem zweiten Quetschventil der Zentrifuge in Eingriff zu gelangen; und
- ein drittes Rohr (4) mit:
- einem ersten Ende, das mit der Spitze des Sammel- und Trennbeutels (1) verbunden ist;
- einem zweiten Ende;
- einen Drei-Wege-Verbinder (16, 16a, 16b) mit:
einem Einlasskanal (20), der mit dem zweiten Ende des dritten Rohrs (4) verbunden ist;
einem ersten Auslasskanal (21), der mit einem zweiten Ende des ersten Rohrs (17) verbunden ist, und
einem zweiten Auslasskanal (22), der mit einem zweiten Ende des zweiten Rohrs (18) verbunden ist,
**dadurch gekennzeichnet, dass** er erste Auslasskanal (21) und der zweite Auslasskanal (22) senkrecht mit dem Einlasskanal (20) verbunden und entlang des Einlasskanals (20) versetzt sind, so dass der erste Auslasskanal (21) weiter vom Ende des Einlasskanals (20), das mit dem dritten Rohr (4) verbunden ist, als der zweite Auslasskanal (22) entfernt ist.

2. Beutelset nach Anspruch 1, wobei sich der erste Auslasskanal (21) an einer ersten Position mit dem Einlasskanal (20) verbindet und sich der zweite Auslasskanal (22) an einer zweiten Position mit dem Einlasskanal (20) verbindet, wobei die erste Position näher an der Drehachse als die zweite Position liegt, wenn der Beutelsatz in der Zentrifuge angebracht ist, wobei der erste Ventilabschnitt mit dem ersten Quetschventil in Eingriff steht und der zweite Ventilabschnitt mit dem zweiten Quetschventil in Eingriff steht.

3. Beutelset nach Anspruch 1 oder 2, wobei das dritte Rohr (4) einen Detektionsabschnitt umfasst, um mit einem Zellendetektor der Zentrifuge in Eingriff zu gelangen.

4. Beutelset nach Anspruch 1, 2 oder 3, das ferner einen bruchfähigen Stopfen (9) umfasst, der mit dem dritten Rohr (4) verbunden ist.

5. Beutelset nach einem der Ansprüche 1 bis 4, wobei die Flüssigkeit aus mehreren Komponenten Vollblut ist, wobei das Beutelset für die Auftrennung von Vollblut in eine Plasmakomponente, eine Blutplättchenkomponente und eine Erythrozytenkomponente vorgesehen ist, wobei der erste Satellitenbeutel (2) zum Sammeln der Plasmakomponente vorgesehen ist und der zweite Satellitenbeutel (15) zum Sammeln der Blutplättchenkomponente vorgesehen ist.

6. Beutelset nach einem der Ansprüche 1 bis 5, das ferner umfasst:
- einen dritten Satellitenbeutel (3);
- ein viertes Rohr (6) mit:
- einem ersten Abschnitt mit einem erste Ende, das mit dem oberen Bereich des Sammel- und Auftrennbeutels (1) verbunden ist; und
- einem zweiten Abschnitt mit einem zweiten Ende, das mit dem dritten Satellitenbeutel (3) verbunden ist; und
- einen Leukozytenverringerungsfilter (13) mit einem Einlass, der mit einem zweiten Ende des ersten Abschnitts des vierten Rohrs (6) verbunden ist, und mit einem Auslass, der mit einem ersten Ende des zweiten Abschnitts des vierten Rohrs (6) verbunden ist.

7. Beutelset nach einem der vorstehenden Ansprüche, das ferner eine Nadel (12) umfasst, die mit einem zweiten Ende des Sammelrohrs (5) verbunden ist.

## Revendications

1. Ensemble de poches pour la séparation d'un liquide composite en au moins deux composants dans une centrifugeuse ayant un axe de rotation, comprenant :
- une poche de collecte et de séparation (1) ayant un axe médian, une partie supérieure et une partie inférieure, où la partie supérieure de la poche de séparation comporte deux bords convergeant vers une pointe située sur l'axe médian ;
- un tube de collecte (5) ayant une première extrémité reliée à la poche de collecte et de séparation (1) ; et
- au moins deux poches satellites (2, 3, 15) reliées à la partie supérieure de la poche de collecte et de séparation (1) ;
- un premier tube flexible (17) ayant :
- une première extrémité reliée à une première poche satellite (2) ;
- une section de valve pour mettre en prise un premier robinet à manchon de la centrifugeuse ;
- un deuxième tube flexible (18) ayant :
- une première extrémité reliée à une deuxième poche satellite (15) ;
- une section de valve pour mettre en prise un deuxième robinet à manchon de la centrifugeuse ; et
- un troisième tube (4) ayant :
- une première extrémité reliée à la pointe de la poche de collecte et de séparation (1)
- une seconde extrémité ;
- un raccord à trois voies (16, 16a, 16b) ayant :
un canal d'entrée (20) relié à la deuxième extrémité dudit troisième tube (4) ;
un premier canal de sortie (21) relié à une seconde extrémité du premier tube (17), et
un second canal de sortie (22) relié à une seconde extrémité du deuxième tube (18),
**caractérisé en ce que** le premier canal de sortie (21) et le second canal de sortie (22) sont reliés perpendiculairement au canal d'entrée (20) et sont décalés le long du canal d'entrée (20) de sorte que le premier canal de sortie (21) soit plus loin que le deuxième canal de sortie (22) de l'extrémité du canal d'entrée (20) qui est relié au troisième tube (4).

2. Ensemble de poches selon la revendication 1, où le premier canal de sortie (21) est relié au canal d'entrée (20) à un premier emplacement et le second canal de sortie (22) est relié au canal d'entrée (20) à un second emplacement, le premier emplacement étant plus proche de l'axe de rotation que le second emplacement lorsque l'ensemble de poches est monté dans la centrifugeuse avec la première section de valve engagée dans le premier robinet à manchon et la deuxième section de valve engagée dans le second robinet à manchon.

3. Ensemble de poches selon la revendication 1 ou 2, où le troisième tube (4) comprend une section de détection pour la mise en prise d'un détecteur de cellule de la centrifugeuse.

4. Ensemble de poches selon la revendication 1, 2 ou 3, comprenant en outre un bouchon cassable (9) relié au troisième tube (4).

5. Ensemble de poches selon l'une quelconque des revendications 1 à 4, où le liquide composite est du sang total, ledit ensemble de poches étant destiné à la séparation du sang total en un composant de plasma, un composant de plaquettes et un composant de globules rouges, où la première poche satellite (2) est destinée à collecter le composant de plasma et la deuxième poche satellite (15) est destinée à collecter le composant de plaquettes.

6. Ensemble de poches selon l'une quelconque des revendications 1 à 5, comprenant en outre :
- une troisième poche satellite (3) ;
- un quatrième tube (6) ayant :
- une première section ayant une première extrémité reliée à la partie supérieure de la poche de collecte et de séparation (1) ; et
- une seconde section ayant une seconde extrémité reliée à la troisième poche satellite (3) ; et
- un filtre de leuco-réduction (13) ayant une entrée reliée à une seconde extrémité de la première section du quatrième tube (6) et une sortie reliée à une première extrémité de la seconde section du quatrième tube (6).

7. Ensemble de poches selon l'une quelconque des revendications précédentes, comprenant en outre une aiguille (12) reliée à une seconde extrémité du tube de collecte (5)
